# EUROPEAN PATENT APPLICATION

(11) **EP 1 579 850 A2**
(43) Date of publication of application: **28.09.2005**
(21) Application number: 05005526.8
(22) Date of filing: 14.03.2005
(51) Int. Cl.: A61K 9/127, A61P 35/00

(54) **A pharmaceutical composition containing liposomes for treating a cancer**

(30) Priority: 15.03.2004 JP 2004073572
(71) Applicant: NIPRO CORPORATION, 531-8510 (JP)
(72) Inventor: Kimura, Toshikiro, Okayama-shi Okayama (DE); Higaki, Kazutaka, Okayama-shi Okayama (DE); Ogawara, Ken-ichi, Okayama-shi Okayama (JP); Kai, Toshiyaka, Osaka-shi Osaka (JP); Yokoe, Jun-ichi, Osaka-shi Osaka (JP); Sakuragi, Shiho, Osaka-shi Osaka (JP); Sato, Makoto, Osaka-shi Osaka (JP)
(74) Representative: Albrecht, Thomas

(57) **Abstract**

The present invention provides liposome-containing drug administration carriers that have improved residence time in the blood and improved migration into tumor cells as compared with conventional ones and that have reduced accumulation in the heart, and a pharmaceutical composition for treating a cancer that includes liposomes containing an antitumor active substance and having polyalkylene glycol and albumin bound thereto and that can be incorporated in large amounts in the cancer cells.

## Description

### TECHNICAL FIELD OF THE INVENTION

The present invention relates to liposomes that have excellent migration in tumor cells and excellent residence time in blood, a pharmaceutical composition for treating a cancer and a method of treating a cancer using the same.

### BACKGROUND ART OF THE INVENTION

Liposomes, which are lipid capsules composed of a lipid double membrane, have been studied for their use as carriers for various medicines, centered on injections.

However, although liposomes are composed of lipids that are biocompatible, they are known to be recognized as foreign substances by immune systems, so that they are incorporated into reticuloendothelial systems represented by the liver, spleen and so on and disappear rapidly from the blood. Therefore, the biggest defect of liposomes is that the action of the drug after administration does not last.

To solve this defect, a method of chemically modifying the surface of liposomes with glycoproteins or glycolipids, a method of binding glucuronic acid derivatives to the surface of liposomes and so on have been reported. However, none of these methods have been used to produce pharmaceutical preparations. On the other hand, in the 1990's, there have been extensive studies on the sustained action of drugs through alleviation of the recognition as a foreign substance by immune systems to improve the residence in blood of liposomes by chemical modification of the surface of the liposomes with a hydrophilic polyethylene glycol.

Liposome preparations have already been developed by application of this technology and amphotericin B, which is an antifungal drug, doxorubicin and daunorubicin, which are anticancer agents, indium, which is a contrast medium, and so on are commercially available (Troy O. Harasym, Marcel B. Bally, Paul Tardi, "Clearance properties of liposomes involving conjugated proteins for targeting" , Advanced Drug Delivery Reviews, 1998, vol. 32, p.99-118). Recently, studies have beenmade on application of this technology to anticancer agents such as cisplatin, vincristine, and camptothecine (Naoto Oku, Yoshihiro Tokudome, Tomohiro Asai and Hideo Tsukada, "Evaluation of Drug Targeting Strategies and LiposomalTrafficking", Current Pharmaceutical Design, 2000, vol.6, p.1669-1691). Also application of this technology to carriers of genes upon gene therapy is being studied.

Currently, doxorubicin hydrochloride is widely used as an antitumor active substance. The substance will disappear rapidly from the blood after intravenous administration because of bile excretion but it is known to cause severe side effects represented by accumulation in the heart. Therefore, when doxorubicin hydrochloride is administered, it has been necessary to closely observe the condition of the patient, for example, by frequently performing clinical tests (blood test, liver function and kidney function tests, and heart function test).

Up to now, there have been commercially available preparations wherein the above-mentioned substance is encapsulated in a liposome with which polyethylene glycol chains are combined for the purpose of improving the residence time in the blood of doxorubicin hydrochloride and avoiding its accumulation in the heart (Alberto Gabizon, Hilary Shmeeda and Yechezkel Barenholz, "Pharmacokinetics of Pegylated Liposomal Doxorubicin", Clinical. Pharmacokinetics, 2003, 42 (5), p.419-436).

Further, surface modification of liposomes has been actively performed also in studies that are contemplated to target drugs to cancer cells or hepatocytes, and a method of binding an antibody or transferrin to liposomes to allow them to recognize cancer cells, a method of binding various sugar chains to liposomes to allow them to be incorporated by hepatocytes, and so on have been reported. In the chemical modification, use of albumin as a spacer has been reported (Shuji Kojima, Yusuke Sogawa, Yoshika Tajiri and Noboru Yamazaki, "Studies on Reduction and Increase of the Reticuloendothelial Systems Uptake of Glycoprotein-conjugated Liposomes by Linking a Terminal Sugar-chain with Sialic Acid", Drug Delivery System, 2002, vol. 17-1, p.63-68).

The present inventors have found that liposomes which contain various physiologically active substances in encapsulated form and with which albumin is combined show improved residence time in the blood (WO 2004-45583A). However, there have been neither reports on studies of a composition containing a liposome combined with albumin, and an antitumor active substance nor reports on studies of incorporation of an antitumor active substance into cancer cells.

The conventional antitumor agents containing liposomes as carriers for medicines do not show enough residence in blood tumor cells and besides show severe side effects. The development of liposomes that show increased migration of antitumor active substances in cancer cells and do not exert severe side effects has been needed.

### SUMMARY OF THE INVENTION

The present inventors have made extensive studies on liposomes preparations. As a result they have found that simultaneously combining a polyethylene glycol (hereinafter, sometimes abbreviated also as "PEG") chain and albumin with liposomes that contain doxorubicin hydrochloride in encapsulated form not only synergistically improves residence time in the blood of liposomes and doxorubicin hydrochloride contained therein in encapsulated form, but also allows both of them to be incorporated into cancer cells in large amounts. Furthermore, the present inventors found that the liposomes inhibit the accumulation of doxorubicin in the heart and migrate into cancer cells in high concentrations, and reduce their heart toxicity and improve the therapeutic effects. Based on these findings, the present inventors continued the studies and accomplished the present invention.

That is, the present invention relates to:
(1) a pharmaceutical composition for treating a cancer, which comprises a liposome combined with a polyalkylene glycol and albumin, and an antitumor active substance, and which can be incorporated in a large amount in cancer cells (hereinafter, sometimes abbreviated as "the liposomes preparation of the present invention");
(2) the pharmaceutical composition according to item (1), wherein the antitumor active substance is an antitumor antibiotic;
(3) the pharmaceutical composition according to item (2), wherein the antitumor antibiotic is an anthracycline antibiotic antitumor agent;
(4) the pharmaceutical composition according to item (3), wherein the anthracycline antibiotic antitumor agent is doxorubicin or a salt thereof;
(5) the pharmaceutical composition according to item (1), wherein the albumin is a genetic recombinant human serum albumin;
(6) the pharmaceutical composition according to item (1), wherein a content of the albumin is about 0.0001 to about 10 mol% based on the total amount of lipid constituting the liposome;
(7) the pharmaceutical composition according to item (1), wherein a volume average particle size of the liposome is about 10 to about 5,000 nm;
(8) the pharmaceutical composition according to item (1), wherein the polyalkylene glycol has a molecular weight of about 200 to about 4,000,000;
(9) the pharmaceutical composition according to item (1), wherein the polyalkylene glycol is polyethylene glycol;
(10) the pharmaceutical composition according to item (1), wherein the composition is an injection agent;
(11)a method of treating a cancer, characterized by parenterally administering the pharmaceutical composition defined in item (1); and
(12)a method of treating a cancer according to item (11), wherein the pharmaceutical composition is parenterally administered.

The liposomes preparation of the present invention not only increases residence time in the blood of the antitumor active substance as compared with those liposomes combined with only polyethylene glycol chains that contain the antitumor active substance in encapsulated form, but also allows the antitumor active substances tomigrate into cancer cells in large amounts . This widens the therapeutic window, and not only a decrease of heart toxicity caused by accumulation of doxorubicin in the heart, which is a side effect, but also a decrease in the dosage of the antitumor active substance and long-sustained pharmacological effects are achieved.

### BRIEF DESCRIPTION OF THE DRAWINGS

Fig.1 is a graph comparing amounts of doxorubicin hydrochloride in blood after administration of the liposomes of Example 1.
Fig. 2 is a graph comparing amounts of doxorubicin hydrochloride in cancer cells after administration of the liposomes of Example 1.

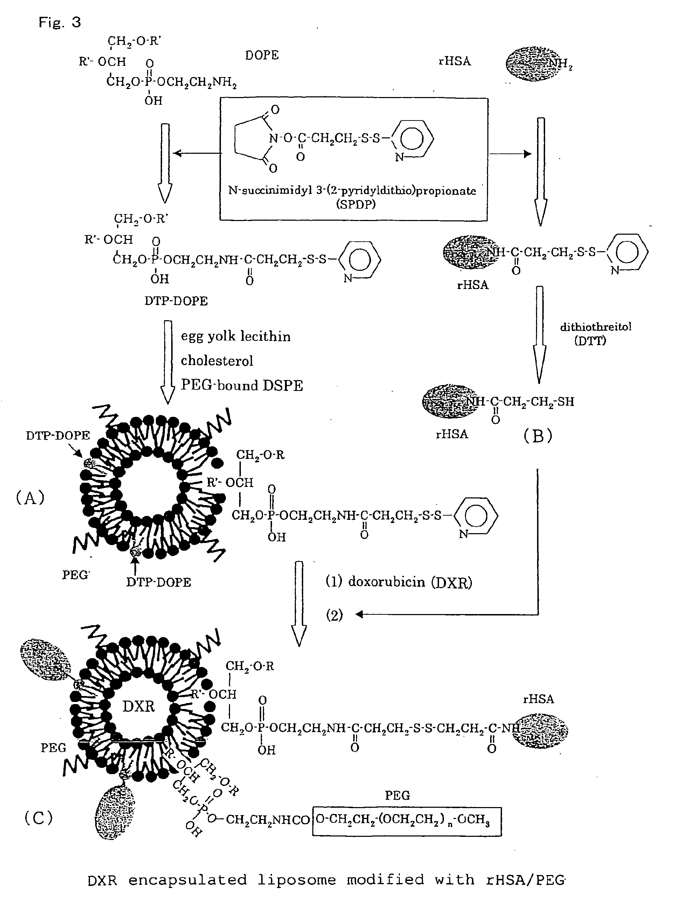

Fig. 3 is a diagram showing a process of producing a pharmaceutical composition containing liposomes as described in Example 1. In Fig. 3, liposomes of diagram (A) wherein R' is an oleoyl group, are produced by using as a constituting lipid, DTP-bonded DOPE obtained by combining DOPE with SPDP, and PEG-bonded DSPE. In diagram (A), only DTP-DOPE is represented by a chemical formula. Doxorubicin hydrochloride(abbreviated as DXR)-encapsulated and PEG-modified liposomes containing DTP-DOPE are produced by incubating doxorubicin in liposomes (A). DXR-encapsulated and rHSA and PEG-modified liposomes represented by diagram (C) , wherein R is stearoyl group, are produced by reacting liposomes (A) with 3-mercaptopropionyl-rHSA represented by diagram (B). In diagram (C), one bond only among bonds between DTP-DOPE and rHSA is represented by a chemical formula, but the other bonds are the same as well. Further only one bond among bonds between DSPE and PEG is represented by a chemical formula, but the other bonds are the same as well.

### DETAILED DESCRIPTION OF THE INVENTION

Usually, the term "liposomes" means closed vesicles composed of lipid molecules that congregate in the form of a membrane with an aqueous phase in the inside thereof (see D.D. Lasic, "liposomes: from basic to applications, " Elsevier Science Publishers, pp. 1-171 (1993)). In the present invention, however, the term means fine particles of lipid that congregate as a whole regardless of whether an inner aqueous phase is contained or not. Further, the structure of liposomes of the present invention is not particularly limited, and may be multi-layered liposomes or single-layer liposomes.

The size of the liposomes in the liposomes preparation of the present invention is not particularly limited; the liposomes in the liposomes preparation of the present invention have a volume average particle size of about 10 to about 5,000 nm, preferably about 50 to about 500 nm. The volume average particle size of the liposomes can be determined based on the principle of dynamic light scattering (see D. D. Lasic, "Liposomes: frombasic to applications," Elsevier Science Publishers, pp. 1-171 (1993)).

A lipid constituting the liposomes in the liposomes preparation of the present invention is not particularly limited, and may be a known lipid. Examples of the above-mentioned lipid include: phospholipids, glycolipids, fatty acids, dialkyl dimethylammonium amphiphiles,polyglycerolalkylether,polyoxyethylene alkylether, and the like (Liposome Technology, 2nd edition, vol.1, 141, 1993); alkylglycoside, alkylmethylglucamide, alkylsucrose ester, dialkyl polyoxyethylene ether, dialkyl polyglycerol ether, and the like (Liposome Technology, 2nd edition, vol.1, 141, 1993); amphiphilic block copolymer such as polyoxyethylene-polylactic acid, and the like (JP 6508831 T); long-chain alkylamines (tetradecyl amine, hexadecyl amine, stearyl amine, and the like); and long-chain fatty acid hydrazides (myristic hydrazide, palmitic hydrazide, stearic hydrazide, and the like).

Examples of the above-mentioned phospholipids include: natural or synthetic phospholipids such as phosphatidylcholine (e.g. soy bean phosphatidylcholine, dilauroyl phosphatidylcholine, dimyristoyl phosphatidylcholine,dipalmitoyl phosphatidylcholine, distearoyl phosphatidylcholine, or the like); phosphatidylethanolamine (e.g.dioleoylphosphatidylethanolamine, dilauroyl phosphatidylethanolamine, dimyristoyl phosphatidylethanolamine, dipalmitoyl phosphatidylethanolamine, distearoyl phosphatidylethanolamine, or the like); phosphatidylserine (e.g. dilauroyl phosphatidylserine, dimyristoyl phosphatidylserine, dipalmitoyl phosphatidylserine, distearoyl phosphatidylserine, or the like); phosphatidic acid; phosphatidylglycerol (e.g. dilauroyl phosphatidylglycerol, dimyristoyl phosphatidylglycerol, dipalmitoyl phosphatidylglycerol, distearoyl phosphatidylglycerol, or the like); phosphatidylinositol (e.g. dilauroyl phosphatidylinositol, dimyristoyl phosphatidylinositol, dipalmitoyl phosphatidylinositol, distearoyl phosphatidylinositol, or the like); and lysophosphatidylcholine; sphingomyelin; egg yolk lecithin; soy bean lecithin; and hydrogenated phospholipids.

Examples of the above-mentioned glycolipids include glyceroglycolipids, sphingoglycolipids, and sterols. Examples of the above-mentioned glyceroglycolipids include: digalactosyl diglycerides (digalactosyl dilauroylglyceride, digalactosyl dimyristoylglyceride, digalactosyl dipalmitoylglyceride, digalactosyl distearoylglyceride, and the like); and galactosyl diglycerides (galactosyl dilauroylglyceride, galactosyl dimyristoylglyceride, galactosyl dipalmitoylglyceride, galactosyl distearoylglyceride, and the like). Examples of the above-mentioned sphingoglycolipids include galactosylcerebroside, lactosylcerebroside, and ganglioside. Examples of the above-mentioned sterols include cholesterol, cholesterol hemisuccinate, 3β-[N-(N', N'-dimethylaminoethane)carbamoyl]-cholesterol, ergosterol, and lanosterol.

In the present invention, the lipids can be used singly or as a combination of two or more thereof.

The polyalkylene glycols that can be used in the present invention are not particularly limited and include those which have an alkylene chain of 1 to 6 carbon atoms. The alkylene chain may optionally be substituted with a substituent that is not detrimental to the present invention, such as a hydroxyl group, a carboxyl group, an amino group, or an alkoxy group. Specifically, for example, polyethylene glycol (PEG), polypropylene glycol and so on can be used. It is particularly preferable that polypropylene glycol be used. The molecular weight of the polyalkylene glycols is not particularly limited and those having a molecular weight of about 200 to about 4,000,000, preferably about 1,000 to about 50,000 can be used. When polyethylene glycol is used, particularly those having the above molecular weight are preferable.

In the present invention, the content of the polyalkylene glycol is not particularly limited but is preferably about 0.5 to about 30 mol% based on the total amount of the lipid that constitutes the liposomes.

The albumins that can be used in the present invention are not particularly limited and include, for example, animal albumins such as egg albumin, serum albumin, milk albumin, and muscle albumin (miogen) and plant albumins such as leucocin, legumelin, and ricin. Among them, it is preferable that serum albumin from the same animal as the target animal that is to be administered, particularly human serum albumin be used in the present invention. Further, the albumins used in the present invention may be albumins that are obtained by genetic engineering techniques. The above-mentioned albumins may have the same amino acid sequence as that of wild type albumin or may be a mutant albumin that has deletion, substitution or addition of one or a plurality of amino acids, preferably one to several amino acids, unless the albumin produces results contrary to the object of the present invention. Such albumins can be prepared with ease by known techniques. In the present invention, preferably the genetically engineered albumins are used since they have no fear of infections.

In the present invention, the content of albumin is not particularly limited and is preferably about 0.0001 to about 10 mol% based on the total amount of lipid.

The liposomes in the liposomes preparation of the present invention can be of any desired structure so far as they include the above-mentioned polyalkylene glycol and albumin. Any position and manner of combining the polyalkylene glycol and albumin may be used. However, the liposomes preferably have the polyalkylene glycol and albumin on their surface. Further, the polyalkylene glycol and albumin may be bonded to the liposomes in any fashion, for example, adsorption, electric bonds, physical bonds such as Van der Waals force, chemical bonds or the like but preferably through chemical bonds.

Preferred modes of the liposomes in the liposomes preparation of the present invention include (a) a mode in which the polyalkylene glycol and albumin, respectively, are bonded to the liposome; (b) a mode in which the liposome and albumin are bonded through the polyalkylene glycol, that is, the liposome is bonded to a site of the polyalkylene glycol and the albumin is bonded to the polyalkylene glycol at a site different from the above-mentioned site; and (c) a mode in which the liposome and polyalkylene glycol are bonded through the albumin, that is, the liposome is bonded to a site of the albumin and the polyalkylene glycol is bonded to the albumin at a site different from the above-mentioned site. In the present invention, liposomes obtained by the modes (a) to (c) may be present in admixture.

The liposomes in the liposomes preparation of the present invention can be produced by using known technologies. Preferable methods (a) to (c) for the production of liposomes will be described separately hereinbelow according to the above-mentioned three embodiments.

### (a) Mode in which the polyalkylene glycol and albumin, respectively, are bonded to the liposome:

The methods of producing the liposomes in the liposomes of the present invention of this mode include:
(i) a method of combining albumin with a liposome to which polyalkylene glycol is bonded;
(ii) a method of combining polyalkylene glycol with a liposome to which albumin is bonded; and
(iii) a method of producing a liposome using a lipid to which polyalkylene glycol is bonded and a lipid to which albumin is bonded.

In the method (i) , the liposome to which polyalkylene glycol is bonded can be produced with ease by using a known method. For example, a method of producing the liposomes using a lipid to which polyalkylene glycol is bonded may be mentioned. Examples of the "lipid to which polyalkylene glycol is bonded" include polyalkylene glycol-modified lipids, polyalkylene glycol alkyl ethers, polyalkylene glycol castor oil derivatives and polyalkylene glycol sorbitan fatty acid esters. The "polyalkylene glycol" moiety of the lipids is preferably polyethylene glycol. The "lipid to which polyalkylene glycol is bonded" is preferably polyethylene glycol-modified phospholipids and, more preferably, one in which the phospholipid is phosphatidylethanolamine.

More specifically, examples thereof include: PEG-DSPE [1,2-distearoyl-sn-glycero-3-phosphatidylethanolamine-N-(polye thylene glycol)]; N-(monomethoxypolyethyleneglycolsuccinyl)-phosphatidylethanolamine represented by the following formula (11):

CH₃- (CH₂CH₂O)ₙ-CO-CH₂CH₂-CO-NH-PE (11)

wherein n is an integer of 5 to 100,000, preferably an integer of 10 to 1,200, and -NH-PE represents a phosphatidylethanolamine residue;
N-[ (monomethoxypolyethyleneglycol)(2-chloro-1,3,5-triazin-4,6-diyl) ]phosphatidylethanolamine represented by the following formula (12): 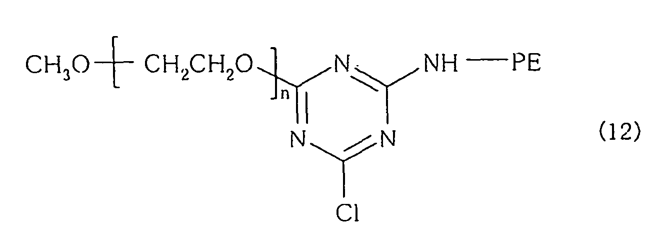 wherein n and -NH-PE have the same meanings as defined above; N-(monomethoxypolyethyleneglycolcarbonyl) phosphatidylethanolamine represented by the following formula (13):

CH₃O- (CH₂CH₂O)ₙ₋₁-CO-NH-PE (13)

wherein n and -NH-PE have the same meanings as defined above; and N-(monomethoxypolyethyleneglycolethylene) phosphatidylethanolamine represented by the following formula (14):

CH₃O-(CH₂CH₂O)ₙ-CH₂CH₂-NH-PE (14)

wherein n and -NH-PE have the same meanings as defined above.

The "lipid to which polyalkylene glycol is bonded" as mentioned above can be produced with ease by using known technologies or a commercially available product may be used. The method of producing liposomes using such lipid as a constituent lipid is not particularly limited and a known method may be used. For example, liposomes can be produced by using the above-mentioned lipid and an aqueous phase, a thin film method, a reversed phase distillation method, an ethanol injection method, an ether injection method, a dehydration-rehydration method, or the like. The volume average particle size of liposomes can be adjusted by an ultrasonic wave irradiation method, an ultrasonic wave irradiation method after freeze-thawing, an extrusion method, a French press method, a homogenization method or the like (see D. D. Lasic, "Liposomes: from basic to applications, Elsevier Science Publishers, pp.1-171 (1993)). Here, the term "aqueous phase" means an aqueous solution that constitutes the inside of liposomes and is not particularly limited so far as it is usually used in this field of the art. Aqueous solutions of sodium chloride, buffer solutions such as a phosphate buffer solution and an acetate buffer solution, sugar solutions such as a glucose solution and a trehalose solution, and a mixed solution thereof are preferable. Generally, in order to maintain the structure of liposomes administered to a living organism in a stable state, the aqueous phase used in the production of the liposomes is preferably nearly isotonic to a body fluid, and the osmotic pressure applied between the inside of liposomes and the body fluid is preferably small.

Combining albumin with the polyalkyleneglycol-bonded liposomes as obtained can be performed with ease by using a known method, for example, a coupling technique of a mercapto group and a maleimide group (sulfhydryl-maleimide coupling technique) (Derksen, J.T.P. and Scherphof, G.L. (1985) Biochem. Biophys. Acta 814, p.151-155) . Among others, a method of combining the liposomes with albumin through a reactive intervening group can be advantageously adopted. The reactive intervening group is not particularly limited and may be any known group in this field of the art.

Preferable modes thereof include the following methods. In case of preparing polyalkyleneglycol-bonded liposomes as described above, in addition to the lipid to which the polyalkylene glycol is bonded, a lipid having a reactive intervening group is used as the lipid. As the lipid having a reactive intervening group, 1,2-dioleoyl-sn-glycero-3-phosphoethanolamine-N-(glutaryl) (hereinafter, abbreviated as "NGPE") is preferable. After preparing polyalkyleneglycol-bonded liposomes as described above using such constituent lipids, albumin is bonded to the liposomes through the reactive intervening groups on the liposomes. When NGPE is used, it is preferable that the amino group of albumin be bonded to the terminal carboxyl group of NGPE. On this occasion, a functional group for increasing the reactivity of the reactive intervening group may be bonded to the liposomes in advance and albumin may be bonded so that albumin substitutes for such functional groups. For example, when NGPE is used, water-soluble carbodiimide is used to combine a carbodiimide group with NGPE in advance and then albumin is bonded to the NGPE such that albumin substitutes for the carbodiimide group.

Other preferable modes include a method wherein 3-(2-pyridyldithio) propionate (hereinafter, abbreviated as "DTP") bound to an amino group of 1,2-dioleoyl-sn-glycero-3-phosphoethanolamine (hereinafter, abbreviated as "DOPE") is used as the lipid (hereinafter, abbreviated as "DTP-DOPE"). Specifically, the polyalkylene glycol-bonded liposomes are prepared as described above using DTP-DOPE in addition to the lipid to which the polyalkylene glycol is bonded as the constituent lipid. On the other hand, a mercapto group is introduced into albumin. The method of introducing a mercapto group is not particularly limited and a known method may be used. Preferably, the albumin in which a mercapto group is introduced can be obtained by introducing DTP into albumin and reacting the resultant with dithiothreitol. By the reaction between the liposomes and albumin, albumin can be bonded to the polyalkylene glycol-bonded liposomes.

The methods (ii) and (iii) described above can be performed with ease according to the descriptions above.

The liposomes preparation of the present invention is produced by the following methods: a method (i) wherein the antitumor agent is carried on the liposomes to which polyalkylene glycol is bonded, followed by combining with albumin; a method (ii) wherein the antitumor agent is carried on the liposomes to which albumin is bonded, followed by combining with polyalkylene glycol; or a method (iii) wherein liposomes are produced from a lipid to which polyalkylene glycol is bonded, a lipid to which albumin is bonded, an antitumor agent, and salt.

The mode of liposomes which carry an antitumor agent is not specially limited, and for example, the antitumor agent may be enclosed within the liposomes, or adsorbed or combined on the surface of the liposomes. Furthermore, the antitumor agent may be adsorbed or combined with the albumin or the polyalkylene glycol. The liposomes which carry an antitumor agent and which are combined with polyalkylene glycol are produced according to per se known methods.

Further the above-mentioned liposomes are produced by contacting liposomes to which polyalkylene glycol is bonded with a solution or suspension containing an antitumor active substance to cause uptake of the antitumor active substance into the liposomes. For example, an aqueous solution or suspension of an antitumor active substace is added to the liposomes to which polyalkylene glycol is bonded, and the resulting mixture is vigorously shaken, stirred, or mixed by ultrasonic waves. After the mixture is uniformly dispersed, it is allowed to stand. In this procedure, an aqueous solution of salt, such as ammonium sulfate may be used in place of water. An antitumor active substance which is not taken into the liposomes is removed by conventional preparation/ purification methods to obtain the obj ect liposomes which carry an antitumor active substance and which are combined with polyalkylene glycol.

The liposomes which carry an antitumor active substance and which are combined with albumin are produced from an antitumor active substance and liposomes to which albumin is bonded by the same procedures as mentioned above.

The liposomes preparation of the present invention is produced by adding an aqueous solution of an antitumor active substance and a salt, e.g. ammonium chloride, to a lipid mixture, e.g. PEG-bound DSPE, DTP-DOPE, egg yolk lecithin or cholesterol, and then heating the resultant to cause hydration, thereby causing the formation of liposomes and uptake of the antitumor active substance into the liposomes at the same time.

After production of the liposomes to which polyalkylene glycol and albumin are bonded, an antitumor active substance may be taken into the liposomes according to the per se known methods.

In the following methods (b) and (c), the same procedures as mentioned above can be adopted.

### (b) Mode in which the liposomes and albumin are bonded through the polyalkylene glycol:

The methods of producing liposomes of the present invention include:
(i) a method of producing liposomes to which polyalkylene glycol is bonded, and combining albumin with the polyalkylene glycol of the obtained liposomes; and
(ii) a method of combining liposomes with the polyalkylene glycol to which albumin is bonded at a site different from the site at which the albumin is bonded.

In the method (i) described above, the method of producing the polyalkylene glycol-bonded liposomes is the same as described above. To combine albumin with the polyalkylene glycol of the liposomes, a known technique may be used. Among others, a technique in which polyalkylene glycol is combined with albumin through a reactive intervening group may be adopted. The reactive intervening group is not particularly limited and may be a known group in this field of the art. A maleimido group is a preferable example of the reactive intervening group.

More specifically, the following method may be mentioned. A reactive functional group is combined with the polyalkylene glycol in the polyalkylene glycol-bonded lipid in advance. For example, it is preferable that a maleimido group be combined with a hydroxyl group of the polyalkylene glycol. By using the resultant lipid, liposomes are produced in the same manner as described above. By reaction of the obtained liposomes with albumin, albumin bonds to the liposomes through the reactive functional group of the polyalkylene glycol that is bonded to the liposomes to give the object liposomes. On this occasion, to facilitate the combining of the reactive functional group with albumin, a known treatment such as introduction of a substituent depending on the reactive functional group may be performed on the albumin in advance. When the reactive functional group is a maleimido group, it is preferable that a mercapto group be introduced into the albumin in advance. The method of introducing a mercapto group is not particularly limited and a known method may be used. More specifically, albumin to which a mercapto group is introduced is obtained by reacting albumin with acetyl thioacetate to combine an acetylthioacetyl group with an amino group of the albumin and then eliminating the acetyl group.

In the method (ii) described above, the method of combining polyalkylene glycol with albumin is not particularly limited and a known technique may be used, but the combining through a reactive intervening group is preferable. The reactive intervening group is not particularly limited and may be a known group in this field of the art. Amaleimido group maybe mentioned as a preferable example. Then, liposomes are combined with the obtained albumin-bonded polyalkylene glycol at a site different from the site at which the albumin is bonded. Also, this method is not particularly limited and a known technique may be used. Preferably, mention may be made of a method in which the polyalkylene glycol to which the lipid is bonded in advance is used to insert the albumin-polyalkylene glycol-lipid complex that is obtained in the preceding step into the liposomes.

More specifically, the polyalkylene glycol is combined with the lipid as described above. Then, a reactive functional group is combined with the polyalkylene glycol in the obtained lipid. For example, it is preferable that a maleimido group be combined with a hydroxyl group of the polyalkylene glycol. Then, albumin is reacted with the polyalkylene glycol in the obtained lipid through the reactive functional group. On this occasion, in order to facilitate the combining of the reactive functional group with the albumin, a known treatment such as introduction of a substituent depending on the reactive functional group may be performed on the albumin. When the reactive functional group is a maleimido group, it is preferable that a mercapto group be introduced into the albumin in advance. The method of introducing a mercapto group is not particularly limited and a known method may be used. More specifically, albumin to which a mercapto group is introduced is obtained by reacting albumin with acetyl thioacetate to combine an acetylthioacetyl group with an amino group of the albumin and then eliminating the acetyl group. On the other hand, liposomes are prepared by a known method in advance and the obtained albumin-polyalkylene glycol-lipid complex is inserted into the liposomes, to thereby obtain the object liposomes.

### (c) Mode in which the liposome and polyalkylene glycol are bonded through the albumin:

The methods of producing the liposomes used for the liposomes preparation of the present invention include:
(i) a method of combining albumin to which polyalkylene glycol is bonded with a liposome at a different site from that at which albumin is bonded to the polyalkylene glycol; and
(ii) a method of producing a liposome to which albumin is bonded, followed by combining polyalkylene glycol with the albumin of the liposome.

In the method (i) described above, the method of combining the polyalkylene glycol with the albumin is not particularly limited and any known method may be used. However, it is preferable that they are bonded through a reactive intervening group. The reactive intervening group is not particularly limited and may be a known group in this field of the art. A preferable example thereof is a maleimido group. More specifically, a reactive functional group is combined with the polyalkylene glycol. For example, it is preferable that a maleimido group be combined with a hydroxyl group of the polyalkylene glycol. Then, albumin is reacted with the polyalkylene glycol through the reactive functional group. On this occasion, in order to facilitate the combining of the reactive functional group with the albumin, a known treatment such as introduction of a substituent depending on the reactive functional group may be performed on the albumin. When the reactive functional group is a maleimido group, it is preferable that a mercapto group be introduced into the albumin in advance. The method of introducing a mercapto group is not particularly limited and a known method may be used. More specifically, albumin to which a mercapto group is introduced is obtained by reacting albumin with acetyl thioacetate to combine an acetylthioacetyl group with an amino group of the albumin and then eliminating the acetyl group.

Then, the thus-obtained albumin to which the polyalkylene glycol is bonded is combined with the liposomes. The method is as described above.

In the method (ii) as described above, the method of combining albumin with liposomes is as described above. Then, polyalkylene glycol is combined with the albumin. The method may also be the same as that described above.

Examples of the above-mentioned production methods include:
a method of combining a liposome including a compound represented by the following formula (1): 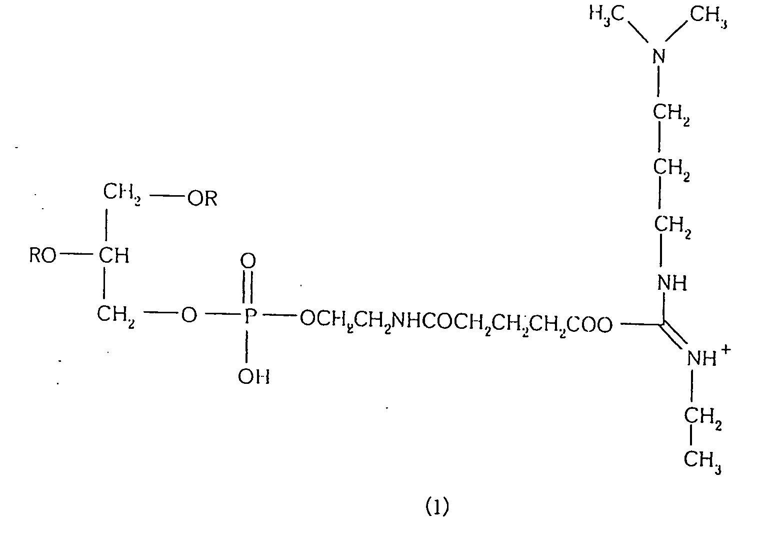 wherein R represents an acyl group derived from a fatty acid having 2 to 35 carbon atoms, as a constituent lipid with albumin;
a method of combining a liposome including a compound represented by the following formula (2): 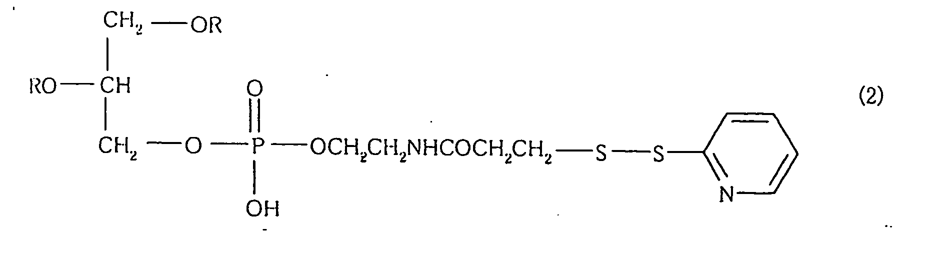 wherein R is as defined above, as a constituent lipid with a compound represented by the following formula (3):

   (Alb-NH)-CO-CH₂-CH₂-SH (3)

   wherein Alb-NH represents albumin and an amino group in the albumin) ;
a method of combining a liposome including a compound represented by the following formula (4): 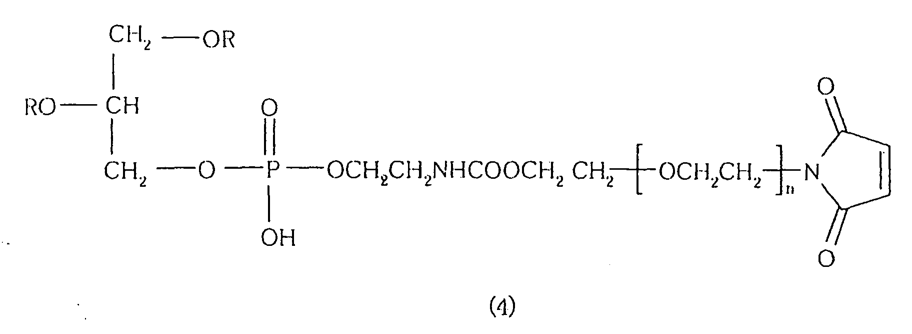 wherein n is an integer of 5 to 100,000, and R is as defined above, as a constituent lipid with a compound represented by the formula (5) :

   (Alb-NH)-CO-CH₂-SH (5)

   wherein Alb-NH is as defined above;
a method of inserting a compound represented by the following formula (6): 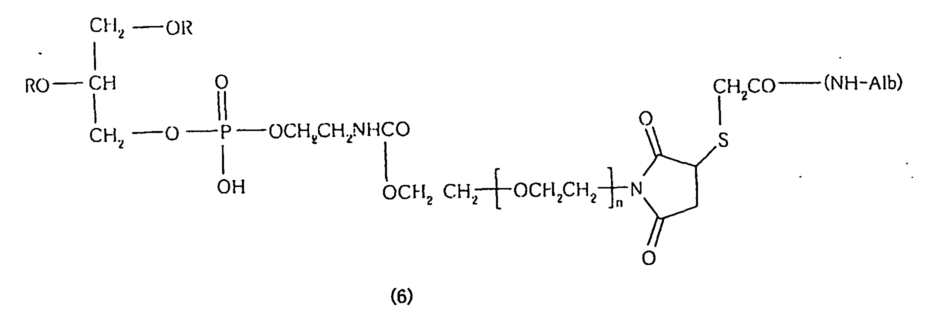 wherein n, R, and Alb-NH are as defined above, into a liposome;
a method of combining a liposome including the compound represented by the formula (1) above as a constituent lipid with a compound represented by the following formula (7): 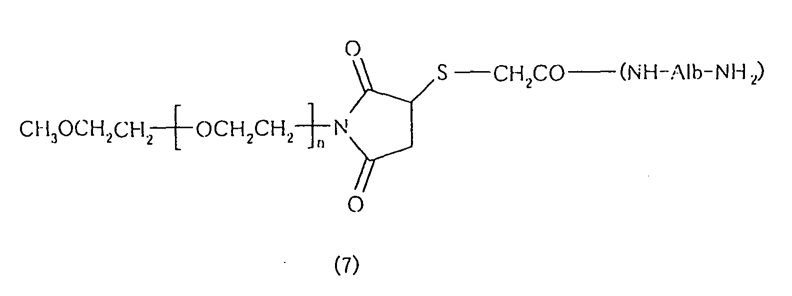 wherein -NH-Alb-NH₂ represents albumin and two different amino groups in the albumin, and n is as defined above; and
a method of combining a liposome including the compound represented by the formula (2) above as a constituent lipid with a compound represented by the following formula (8): 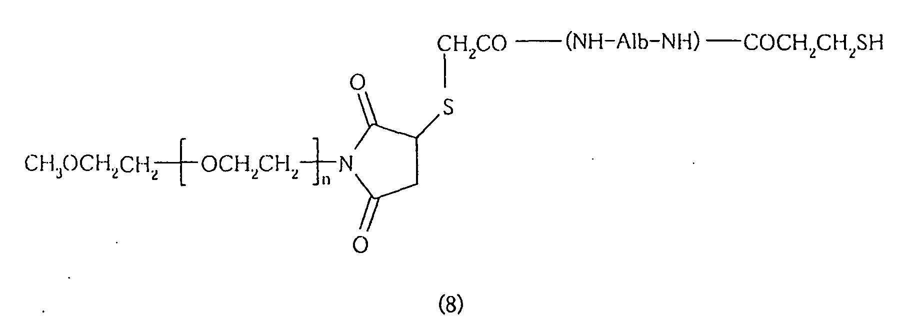 wherein -NH-Alb-NH- represents albumin and two different amino groups in the albumin, and n is as defined above.

In the production method as mentioned above, liposomes as a raw material are used preferably after an antitumor active substance is carried thereon. For example, the antitumor active substance may be encapsulated in the liposomes or may be adsorbed or bonded to the surface of the liposomes. Alternatively, the antitumor active substance may be adsorbed or bonded to the albumin or polyalkylene glycol.

The antitumor active substance is not particularly limited and may be any compound or substance composition so far as it can be administered to animals, preferably humans.

The antitumor active component is not particularly limited, examples of which include antitumor antibiotics, alkylating agents, various antimetabolites, other antitumor agents, antitumor plant components, BRM (biological response modifiers), inhibitors of angiogenesis, cell adhesion inhibitors, matrix metalloprotease inhibitors, and hormones. Of these, antitumor antibiotics are preferably used.

More specific examples of the antitumor antibiotics include: anthracycline antibiotic antitumor agents such as mytomycin C, bleomycin, peplomycin, daunorubicin, aclarubicin, doxorubicin, pirarubicin, THP-adriamycin, 4'-epidoxorubicin, and epirubicin; chromomycin A3; actinomycin D; and salts or complexes thereof. Of these, anthracycline antibiotic antitumor agents are preferably used. More preferably doxorubicin or an acid addition salt thereof (e.g. hydrochloride) is used.

Examples of the other antitumor agents include cisplatin, carboplatin, tamoxifen, camptothecin, ifosfamide, cyclophosfamide, melphalan, L-asparaginase, aceglatone, sizofiran, picibanil, ubenimex, and krestin, and salts or complexes thereof. Further, procarbazine, pipobroman, neocarzinostatin, and hydroxyurea can be given.

Examples of the alkylating agents include: alkylating agents such as nitrogen mustard, nitrogen mustard N-oxide, and chlorambucil; aziridine alkylating agents such as carboquone and thiotepa; epoxide alkylating agents such as dibromomannitol and dibromodulcitol; nitrosourea alkylating agents such as carmustine, lomustine, semustine, nimustine hydrochloride, streptozotocin, chlorozotocin, and ranimustine; busulfan; improsulfan tosylate; and dacarbazine.

Examples of the various antimetabolites include: purine antimetabolites such as 6-mercaptopurine, 6-thioguanine, and thioinosine; pyrimidine antimetabolites such as fluorouracil, tegafur, tegafur/uracil, carmofur, doxifluridine, broxuridine, cytarabine, and enocitabine; folic acid antimetabolites such as methotrexate and trimethotrexate; and salts or complexes thereof.

Examples of the antitumor plant components include: vinca alkaloids such as vindesine, vincristine, and vinblastine; epipodophyllotoxins such as etoposide and teniposide; and salts or complexes thereof.

Examples of the BRM include tumor necrosis factors, indometacin, and salts or complexes thereof.

Examples of the inhibitors of angiogenesis include fumagillol derivatives, and salts or complexes thereof. Examples of the cell adhesion inhibitors include substances each having RGD sequence, and salts or complexes thereof.

Examples of the matrix metalloprotease inhibitors include marimastat, batimastat, and salts or complexes thereof.

Examples of the hormones include hydrocortisone, dexamethasone, methylprednisolone, prednisolone, prasterone, betamethasone, triamcinolone, oxymetholone, nandrolone, metenolone, fosfestrol, ethinylestradiol, chlormadinone, and medroxyprogesterone, and salts or complexes thereof.

The liposomes preparation of the present invention may consist of the liposomes alone that carry the above-mentioned antitumor active substance, but usually they are produced by mixing the liposomes and pharmacologically acceptable carriers by a method known per se (e.g., a conventional method in the field of producing preparations, for example, the method described in Japan Pharmacopoeia (for example, 13th revision)). As the pharmacologically acceptable carriers, various conventional organic or inorganic carrier materials are used as raw materials for preparations. Examples thereof include: excipients, lubricants, binders, disintegrants in solid preparations; and solvents, dissolution auxiliaries, suspending agents, isotonic agents, buffers, soothing agents in liquid preparations. Further, preparation additives such as surfactants, foaming agents, dyes, acidulants, antiseptics, antioxidants, colorants, sweeteners, and flavoring substances may be used as necessary.

More specific examples of the pharmacologically acceptable carriers in solid preparations include: inorganic salt excipients such as calcium citrate and calcium phosphate; lubricants such as magnesium stearate, calcium stearate, light silicic acid anhydride, and hydratedsilicon dioxide;binderssuch as hydroxpropylcellulose, hydroxypropylmethylcellulose, pregelatinized starch, polyvinyl alcohol, polyvinylpyrrolidone, gum arabic powder, gelatin, and pullulan; celluloses such as hydroxypropylcellulose of low-substitution and crystalline cellulose; various starches and starch derivatives such as corn starch, partially α-starch, and hydroxypropyl starch; and disintegrants such as crosspovidone and bentonite.

Examples of the pharmacologically acceptable carriers in liquid preparations include: solvents such as a salt solution, a glucose solution, and a mixture of a salt solution and a glucose solution; dissolution auxiliaries such as dextran, polyvinylpyrrolidone, sodium benzoate, ethylenediamine, salicylamide, nicotinamide, and polyoxyethylene hardened castor oil derivatives; buffers such as a borate buffer, a phosphate buffer, a citrate buffer, a tartrate buffer, and an acetate buffer; polyalcohols such as albumin, glycerin, and propylene glycol; and soothing agents such as lidocaine hydrochloride and benzyl alcohol.

Examples of the preparation additives include: surfactants such as sorbitan fatty ester, polyoxyethylene fatty ester, phospholipid, glycerin fatty ester, polyethylene glycol fatty ester, polyoxyethylene hardened caster oil, polyoxyethylene alkyl ether, and sucrose fatty ester; foaming agents such as sodium hydrogen carbonate, sodium carbonate, and calcium carbonate; acidulantssuch as citric acid, tartaric acid, and malic acid; dyes such as red iron oxide, yellow iron oxide, and tar dyes; fragrances such as lemon, lemon-lime, orange, pineapple, mint, and menthol; sweeteners such as sodium saccharin, dipotassium glycyrrhizinate, aspartame, stevia, and thaumatin; and flavoring substances such as citric acid, sodium citrate, succinic acid, tartaric acid, fumaric acid, and glutamic acid.

Further, examples of stabilizing agents include saccharides and sodium sulfite. Examples of the saccharides include: monosaccharides such as glucose, fructose, xylitol, fucose, and galactose; disaccharides such as maltose, sucrose, lactose, lactulose, and melibiose; oligosaccharides such as fructooligosaccharide, galactooligosaccharide, and lactooligosaccharide; and polysaccharides such as dextran.

Examples of preservatives include ester of paraoxybenzoic acid, benzyl alcohol, chlorocresol, phenethyl alcohol, and benzethonium chloride.

Examples of chelators include sodium edetate and sodium citrate.

Examples of the antioxidants include sodium sulfite, sodium hydrogen sulfite, sodium ascorbate, and sodium thiosulfate.

Examples of a dosage form of the liposomes preparation of the present invention include: oral preparations such as tablets, capsules (including soft capsules, microcapsules, and enteric-coated capsules), powders, granules, and syrups; parenteral preparations such as injections (for example, subcutaneous injections, intravenous injections, intramuscular injections, and intraperitoneal injections), external preparations (for example, transnasal preparations, percutaneous preparations, and ointments) , suppositories (for example, rectal suppositories and vaginal suppositories), pellets, drops, and sustained-release preparations (forexample,sustained-release microcapsules). It is particularly preferable that the liposomes preparation of the present invention takes a dosage form of an injection.

A dose of the liposomes preparation of the present invention may vary depending on the kind of the antitumor active substance that the liposomes have, the dosage formof the liposomes preparation, the kind of disease to be treated, severity of the symptom and disease, age, sexuality or body weight of the patient, administration method and so on and cannot be determined uniformly but may be determined by doctors by comprehensively taking into consideration the above-mentioned conditions.

An administration route of the liposomes preparation of the present invention is not particularly limited and may be an oral administration or a parenteral administration depending on the form of the liposomes preparation of the present invention as described above. Parenteral administration is preferable. When the liposomes preparation of the present invention is an injection, examples of administration forms that are medically suitable include intravenous injection, subcutaneous injection, intracutaneous injection, intramuscular injection, and intraperitoneal injection.

The liposomes preparation of the present invention can be used for the prevention or treatment or therapy of various diseases depending on the kind of antitumor active substance carried by the liposomes preparation of the present invention. For example, the liposomes preparation of the present invention is useful for the prevention or therapy of tumors such as large bowel cancer, brain tumor, head and neck tumor, breast cancer, lung cancer, esophagus cancer, stomach cancer, liver cancer, gallbladder cancer, bile duct cancer, pancreatic cancer, pancreas islet cell cancer, choriocarcinoma, colon cancer, renal cell carcinoma, adrenocortical cancer, urinary bladder cancer, testis cancer, prostate cancer, orchioncus, ovary cancer, uterus cancer, thyroid cancer, malignant carotenoid tumor, skin cancer, malignant melanoma, osteosarcoma, soft tissue sarcoma, neuroblastoma, Wilm's tumor, retinoblastoma, melanoma, and squamous cell cancer.

The liposomes preparation of the present invention can not only increase residence time in the blood, but also can migrate into cancer cells in large amounts. The liposomes preparation of the present invention can be used for treating a cancer advantageously and not only an alleviation of cardiotoxicity (e.g., accumulation of doxorubicin and the like in the heart) which is a side effect but also a decrease in the dose of antitumor active substance and a long-sustained pharmacological effect are obtained.

Hereinafter, the present invention will be described in detail based on examples. rHSA used in the following examples was obtained from BIPHA CORPORATION, Japan. Note that abbreviations described in the examples below are defined as follows.
- DOPE:: 1,2-dioleoyl-sn-glycero-3-phosphoethanolamine
- DSPE:: 1,2-distearoyl-sn-glycero-3-phosphoethanolamine
- DTP:: 3-(2-pyridyldithio)propionyl
- DTT:: dithiothreitol
- DXR:: doxorubicin hydrochloride
- EDTA:: ethylenediamine tetraacetic acid sodium salt
- HEPES:: N-2-hydroethylpiperazine-N'-2-ethanesulfonic acid
- NGPE:: 1,2-dioleoyl-sn-glycero-3-phosphoethanolamine-N-(glutaryl)
- NHS:: N-hydroxysuccimide
- PBS:: phosphate buffered solution of pH 7.4 (obtained from sodium chloride, potassium chloride, potassium dihydrogen phosphate and disodium hydrogen phosphate)
- PEG:: polyethylene glycol
- rHSA:: recombinant human serum albumin
- SATA:: N-succinimidyl-S-acetylthioacetate
- SPDP:: N-succinimidyl 3-(2-pyridyldithio)propionate
- WSC:: water soluble carbodiimide, i.e., 1-ethyl-3-(3-dimethylaminopropyl)carbodiimide

### Example 1

### (1) Production of phospholipid (DTP-DOPE)

12 µmol of SPDP (manufactured by Pierce Biotechnology, Inc. U.S.A.) were added to 0.34 ml of a solution of 10 µmol of DOPE in chloroform and the resultant mixture was stirred for 2 hours. 2 ml of PBS were added to the reaction mixture and the mixture was shaken vigorously for 5 minutes and then centrifuged at 3,000 rpm for 10 minutes. A PBS layer was removed, and purified water was added to a chloroform layer. The mixture was vigorously shaken for 3 minutes, followed by removal of an aqueous layer again by centrifugation. Washingthethus-obtained chloroform solution with the purified water was performed once again. A white semi-solid remaining in a lower layer was subjected to distilling off the solvent and drying for 90 minutes by an evaporator. 1.0 ml of chloroform was added to the residue to dissolve it, thereby to produce a solution of DTP-bonded DOPE in chloroform.

### (2) Production of PEG-modified liposomes containing DTP-DOPE

Lipid (total lipid: 115 µmol, egg yolk lecithin (manufactured by Asahi Kasei Corporation, Japan): cholesterol (manufactured by Wako Pure Chemical Industries, Ltd., Japan): PEG-bound DSPE (manufactured by Avanti Polar Lipids, Inc. U.S.A.) at a ratio of 62 : 33 : 5) were dissolved in chloroform and the resulting mixture was added in an eggplant-shaped flask. Further, a solution of 4.8 µmol of DTP-DOPE in chloroform as obtained above was added thereto and then chloroform was added to become about 7 ml of solution. Thereafter, the solvent was distilled off under reduced pressure by a rotary evaporator and the residue was dried overnight. 6.0 ml of an aqueous solution containing 250 mM of ammonium sulfate were added to the resulting lipid thin film, which was hydrated while heating to 60°C with applying a vortex. Further, sizing was performed by using an extruder so that the particle size was reduced to 100 nm. An aqueous ammonium sulfate suspension of PEG-modified liposomes containing DTP-DOPE was obtained.

### (a) Measurement of particle size distribution of liposomes

Particle size: ca.100 mn (NICOMP370ZLS, Particle Sizing System Co., U.S.A.)

### (b) Measurement of the amount of PEG-modification on the surface of liposomes

### Presence of PEG on the surface of liposomes: 3.5% (quantitative analysis based on picrate method)

Quantitative analysis based on a picrate method was conducted according to the method as described in International Journal of Pharmaceutics, 203, pp. 255-263 (2000). To 10 ml of liposomes were added 20 ml of sodium nitrate-picric acid solution and the resultant was mixed. To the mixture, 10 ml of 1,2-dichloroethane were added and mixed well (extraction), and then the resultant was centrifuged at 1500 g. The organic layer was separated and the absorbance at 378 nm was measured. 70% of overall PEG was confirmed to be present on the surface, since 4.01 µmol of PEG were present on the surface per 5.75 µmol of the employed PEG lipid, from the absorbance at 378 nm.

### (3) Encapsulation of DXR in liposomes

An aqueous ammonium sulfate suspension of the PEG-modified liposomes containing the DTP-DOPE was subjected to gel-filtration (Sepharose CL-6B, Bio-Rad Co., Column: inside diameter 1. 5 cm × 30 cm, Eluting agent: PBS) to obtain an aqueous PBS suspension of the PEG-modified liposomes containing the DTP-DOPE.

To the suspension were added 3. 5 mg of DXR and then the mixture was subjected to incubation at 65°C for 1 hour. Again, the gel filtration was performed in the same way as mentioned above to remove non-encapsulated DXR, and DXR-encapsulated liposomes were collected to obtain DXR-encapsulated and PEG-modified liposomes containing DTP-DOPE. In the gel filtration (Sepharose CL-6B, Bio-Rad Co.), separation and purification based on the difference of molecular weight was conducted. First, doxorubicin encapsulated in liposomes and then doxorubicin unenclosed was eluted. Since doxorubicin shows red-fluorescence, the presence of doxorubicin encapsulated in liposomes was judged by the eye and was further confirmed by using HPLC method (Fluorescence detector RF-10A, Pump LC-10AS, Shimazu Co., Japan).

### (4) Production of 3-mercaptopropionyl-rHSA

13 µmol of SPDP was added to an aqueous solution of 0.67 µmol of rHSA (manufactured by BIPHA CORPORATION, Japan), and the mixture was stirred to produce DTP-rHSA. Then, gel filtration (Sepharose CL-6B, Bio-Rad Co., Eluting agent: Acetate buffer solution of pH 4.5) was performed to separate a DTP-rHSA fraction.

DTT (manufactured by KANTO KAGAKU Co., Japan) was added to DTP-rHSA to become a final concentration of 50 mM, and the resultant mixture was stirred for 20 minutes to convert 2-pyridyldithio group to mercapto group on DTP of DTP-rHSA. To remove unreacted DTT, gel filtration (Sepharose CL-6B, Bio-Rad Co., Eluting agent: PBS of pH 8 . 0) was performed to separate 3-mercaptopropionyl rHSA fraction.

Since 2-thiopyridone (2-TP) which is by-produced by reaction of DTP-rHSA with DTT shows a specific peak of Absorbance at 343 nm, Absorbance of the reaction mixture was measured by spectrophotometer (U-3300, Hitachi Co., Japan). Absorbance of the reaction mixture was by about 0.5 bigger than that of the solution of DTP-rHSA of starting material. Accordingly the formation of 2-TP by reaction of DTP-rHSA with DTT was confirmed.

### (5) Production of the DXR-encapsulated liposomes modified with rHSA and PEG

The 3-mercaptopropionyl-rHSA solution was added to the PEG-modified and DXR-encapsulated liposomes containing DTP-DOPE and the mixture was stirred at room temperature for about 24 hours, thereby to modify the liposomes with rHSA. Thereafter, the reaction mixture was subjected to gel filtration (Sepharose CL-6B, Bio-Rad Co. , Eluting agent: PBS of pH 8. 0) to separate liposomes and unreacted rHSA, followed by collecting the liposome fraction to obtain the DXR-encapsulated liposomes modified with rHSA and PEG.

By electrophoresis analysis using SDS-polyacrylamide of the product, the product was confirmed to be the object DXR-encapsulated liposomes modified with rHSA and PEG, since a band appeared at the position of rHSA monomer.

Particle size was measured by the same method as mentioned in above (2) (a), and was confirmed to be about 100 nm.

### Test Example 1 DXR concentration in plasma

Cancer cells (rat ascites liver cancer) were transplanted to three rats and 6 mg/kg (DXR amount) of the liposome sample prepared in Example 1 was administered to each rat from a tail vein. After a predetermined time period, about 200 µl of blood were collected from the carotid artery, which was immediately centrifuged (at 4°C or less and 1,500 × g, for 5 minute) . 100 µl of a supernatant were collected, which was mixed with 900 µl of an aqueous saturated solution of ammonium sulfate, followed by addition of 2 ml of a mixed solution chloroform/2-propanol = 1:1 and shaking for 10 minutes. This sample was centrifuged at 1,500 × g for 10 minutes to collect an oil layer. The oil layer was subjected to solvent distillation by a rotary evaporator and then the residue was dissolved again in a moving phase. Liquid chromatographic measurement was performed (SCL-10A, manufactured by Shimadzu Co, Japan).

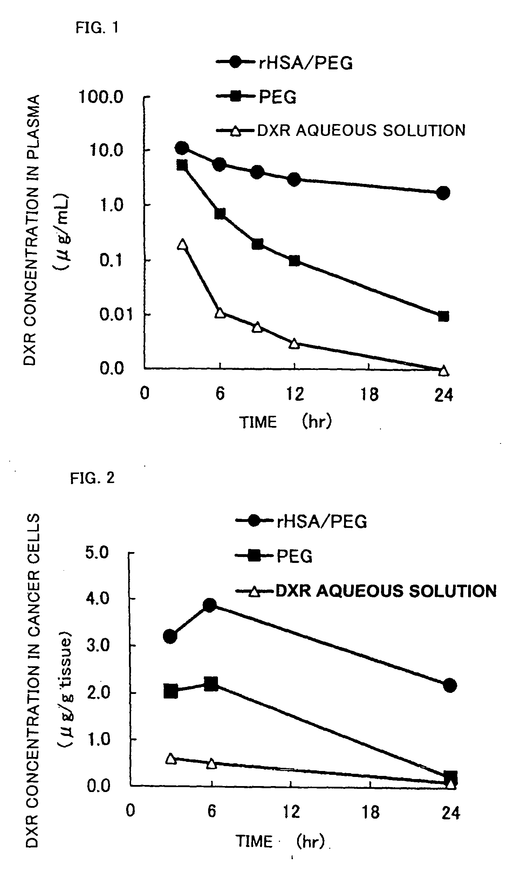

For comparison, the experiments were repeated using the DXR-encapsulated liposomes modified with PEG and an aqueous solution of DXR (800µg/ml). Fig. 1 shows the results.

Fig. 1 clearly indicates that the DXR-encapsulated liposomes modified with rHSA/PEG in Example 1 shows significantly improved residence in plasma as compared with that of the DXR-encapsulated liposomes modified with PEG alone and with that of the aqueous solution of DXR.

### Test Example 2 DXR concentration in cancer cell

The rats from which blood was collected in Test Example 1 above were immediately sacrificed by bleeding to death. After the bleeding, the tumors were collected from the rats for weighing, and each was homogenized in 2.0 ml of PBS. 1 ml of the mixture was collected, which was mixed with 3.0 ml of an aqueous saturated solution of ammonium sulfate, followed by addition of 6 ml of a mixed solution of chloroform/2-propanol = 1 : 1 and shaking for 10 minutes. Each sample was centrifuged at 1, 500 × g for 10 minutes and the oil layer was collected. Each oil layer was subjected to solvent evaporation by a rotary evaporator and then dissolved again in the moving phase, followed by liquid chromatographic measurement (SCL-10A, manufactured by Shimadzu Co. Japan).

For comparison, the same experiments were repeated except that the DXR-encapsulated liposomes modified with PEG and an aqueous solution of DXR (800µg/ml) were used. Fig. 2 shows the results.

Fig. 2 clearly indicates that the DXR-encapsulated liposomes modified with rHSA/PEG in Example 1 show significantly improved migration in cancer cell as compared with that of the DXR-encapsulated liposomes modified with PEG alone and with that of the DXR aqueous solution.

## Claims

1. A pharmaceutical composition for treating a cancer, which comprises a liposome combined with a polyalkylene glycol and albumin, and an antitumor active substance, and which can be incorporated in a large amount in cancer cells.

2. The pharmaceutical composition according to claim 1, wherein the antitumor active substance is an antitumor antibiotic.

3. The pharmaceutical composition according to claim 2, wherein the antitumor antibiotic is an anthracycline antibiotic antitumor agent.

4. The pharmaceutical composition according to claim 3, wherein the anthracycline antibiotic antitumor agent is doxorubicin or a salt thereof.

5. The pharmaceutical composition according to claim 1, wherein the albumin is a genetic recombinant human serum albumin.

6. The pharmaceutical composition according to claim 1, wherein a content of the albumin is about 0.0001 to about 10 mol% based on the total amount of lipid constituting the liposome.

7. The pharmaceutical composition according to claim 1, wherein a volume average particle size of the liposome is about 10 to about 5,000 nm.

8. The pharmaceutical composition according to claim 1, wherein the polyalkylene glycol has a molecular weight of about 200 to about 4,000,000.

9. The pharmaceutical composition according to claim 1, wherein the polyalkylene glycol is polyethylene glycol.

10. The pharmaceutical composition according to claim 1, wherein the composition is an injection agent.

11. The use of a liposome combined with a polyalkylene glycol and albumin, and an antitumor active substance for the preparation of a medicament for treating cancer.

12. The use of claim 11, wherein the medicament is adapted for parenteral administration.
